# EUROPEAN PATENT APPLICATION

(11) **EP 1 726 309 A1**
(43) Date of publication of application: **29.11.2006**
(21) Application number: 06252714.8
(22) Date of filing: 25.05.2006
(51) Int. Cl.: A61K 36/746

(54) **Inhibition of histone deacetylase and of tumor necrosis factor converting enzyme**

(30) Priority: 26.05.2005 US 684742 P; 22.05.2006 US 438547
(71) Applicant: Morinda, Inc., Provo, UT 84604 (US)
(72) Inventor: Palu, Afa Kehaati, American Fork, UT 84003 (US); Zhou, Bing-Nan, Pleasant Grove, UT 84062 (US); Jensen, Claude Jarakae, Cedar Hills, UT 84062 (US); Story, Stephen, Alpine, UT 84004 (US)
(74) Representative: Wilson, Peter David

(57) **Abstract**

The present invention relates to methods and compositions for inhibiting Histone Deacetylase ("HDAC") and/or Tumor Necrosis Factor Converting Enzyme ("TACE") in living organisms. More particularly, the present invention relates to methods and compositions involving the inhibition of HDAC and/or TACE using processed *Morinda citrifolia L.* plant products.

## Description

### Background Of The Invention

### Related Applications

This application claims priority to United States Provisional Application Serial No. 60/684,742, filed May 26, 2005, and entitled, "Histone Deacetylase and Tumor Necrosis Factor Converting Enzyme Inhibition," which is incorporated by reference in its entirety herein.

### Field of the Invention

The present invention relates to methods and compositions for inhibiting Histone Deacetylase ("HDAC") and Tumor Necrosis Factor Converting Enzyme ("TACE") in living organisms. More particularly, the present invention relates to methods and compositions involving the inhibition of HDAC and TACE using processed *Morinda citrifolia L.* plant products.

### Background Information

Histone Deacetylase ("HDAC") and Tumor Necrosis Factor Converting Enzyme ("TACE") have been implicated in various human diseases including Huntington's disease and Rheumatoid arthritis. Significant research has been devoted to preventing and ameliorating such conditions and related effects.

Huntington's disease (HD) is a progressive, fatal neurodegenerative disease caused by a mutation in the huntingtin gene. Mutant huntingtin affects gene expression. There is no known cure or treatment that significantly affects disease progression. However, although the sequence of events leading to neuronal death in HD is unknown, it is possible that interference with gene transcription leading to disruption of normal gene expression could be an important step.

Gene transcription is regulated by complex interactions between proteins and histones and by the modification of these molecules via acetylation, methylation and phosphorylation. Mutant huntingtin reduces histone acetylation by binding to histone acetyltransferase and can thereby affect gene transcription. Drugs that prevent histone deacetylation can restore transcription in the presence of mutant huntingtin. These drugs, known as histone deacetylase (HDAC) inhibitors, are being studied as cancer chemotherapy agents and include sodium butyrate, phenylbutyrate, trichostatin A, and suberoylanilide hydroxamic acid (SAHA)

For example some research has shown that sodium butyrate, an HDAC inhibitor, significantly enhances survival and reduces neuropathological effects, as well as motor deficits, of HD in R6/2 transgenic mouse models. Sodium butyrate and similar HDAC inhibitors are currently being tested as anticancer drugs due to their inhibitory effects in cell proliferation models.

Rheumatoid arthritis is one of the more common autoimmune diseases. An estimated 5 million individuals suffer from rheumatoid arthritis, a figure which will increase to 5.7 million by 2010. Rheumatoid arthritis is a chronic syndrome
characterized by non-specific, usually symmetrical inflammation of the peripheral joints, manifested by the formation of hypertrophied synovia known as panni. Pannus formation mirrors the destruction of articular and peri-articular structures, with or without generalized manifestations. The condition differs from osteoarthritis not only through the obligatory involvement of the immune system but also because disease onset occurs early on in life, generally between the ages of 20 and 50, although it can begin at any age. The inflammation involves the activation of T cells that subsequently infiltrate the synovium. Prolonged inflammation leads to pannus formation and the release of auto-destructive mediators.

HDAC inhibitors have been linked to a possible approach to rheumatoid arthritis. For example, recent research has demonstrated that the inhibition of HDAC7 expression causes increased T cell apoptosis in response to TCR activation. In other research two different HDAC inhibitors inhibited histone H3 and H4 acetylation in synovial fibroblasts. This led to reduced proliferation, an effect which was greater in fibroblasts derived from rats with experimental rheumatoid arthritis than controls. In further in vivo studies continuous topical administration of HDAC inhibitors was found to locally reduce paw swelling by up to 50% in rats. This effect was longer lasting than when oral hydrocortisone was administered and was mirrored by histological improvement; the expression of cell cycle inhibitors such as p16 INK4 and p21 Cip1 and; the reduced expression of TNF alpha, a cytokine which is central to the inflammatory process in rheumatoid arthritis.

### SUMMARY OF THE INVENTION

Embodiments of the present invention comprise methods and compositions for inhibiting Histone Deacetylase ("HDAC") and/or Tumor Necrosis Factor Converting Enzyme ("TACE") without causing negative side effects of known HDAC and/or TACE inhibitors.

Some embodiments comprise *Morinda citrifolia* compositions, each of which includes one or more processed *Morinda citrifolia L.* products. The *Morinda citrifolia* product preferably includes *Morinda citrifolia* fruit juice, which juice is preferably present in an amount capable of maximizing the inhibition of the HDAC and/or TACE without causing negative side effects when the composition is administered to a mammal.

Some embodiments of methods of the present invention comprise the administration and/or consumption of *Morinda citrifolia* extracts in amounts that inhibit HDAC and/or TACE in mammals. Methods of the present invention also include the obtaining of *Morinda citrifolia* compositions and extracts, including *Morinda citrifolia* fruit juice and concentrates thereof.

Some embodiments provide methods of inhibiting the activity of the HDAC and/or TACE without causing the negative secondary effects caused by known HDAC and/or TACE.

Some embodiments provide an orally administered HDAC and/or TACE inhibitor capable of use during pregnancy.

Some embodiments provide an orally administered composition capable of inhibiting HDAC and/or TACE activity in patients that do not respond to known HDAC and/or TACE inhibitors.

Some embodiments provide an over-the-counter composition for inhibiting HDAC and/or TACE activity in mammals without requiring a prescription.

Some embodiments comprise methods and/or compositions for treating mammals comprising administering a formulation containing at least one processed *Morinda citrifolia* product present in an amount by weight between about 0.1 and 99 percent, wherein the formulation is adapted to affect a mammal in a way comprising: inhibiting HDAC and/or TACE, preventing a complication of a primary disorder in patients wherein said complication results from HDAC and/or TACE activity, treating a primary disorder in patients wherein said disorder results from HDAC and/or TACE activity, preventing a primary disorder in patients wherein said disorder results from HDAC and/or TACE activity.

### DETAILED DESCRIPTION OF THE INVENTION

The following description of embodiments of the methods and compositions of the present invention is not intended to limit the scope of the invention, but is merely representative of some embodiments, including the preferred embodiments, of the present invention.

The present invention comprises compositions and methods for inhibiting HDAC and/or TACE activity in mammals, including humans, and compositions and methods for treating an preventing Huntington's disease and Rheumatoid arthritis and related secondary conditions.

The present invention comprises *Morinda citrifolia* compositions, each of which include one or more processed products from the *Morinda citrifolia L.* plant. The *Morinda citrifolia* products preferably include *Morinda citrifolia* fruit juice, which juice is preferably present in an amount capable of maximizing the inhibition of HDAC and/or TACE without causing negative side effects when the composition is administered to a mammal. Extracts of the *Morinda citrifolia* plant may include one more parts of the *Morinda citrifolia L.* plant, including but not limited to the: fruit, including the fruit juice and fruit pulp and concentrates thereof, leaves, including leaf extract, seeds, including the seed oil, flowers, roots, bark, and wood.

Some compositions of the present invention comprise *Morinda citrifolia* extracts present between about 1 and 5 percent of the weight of the total composition. Other such percentage ranges include: about .1 and 50 percent; about 85 and 99 percent; about 5 and 10 percent; about 10 and 15 percent; about 15 and 20 percent; about 20 and 50 percent; and about 50 and 100 percent.

In some *Morinda citrifolia* compositions of the present invention, *Morinda citrifolia* fruit juice evaporative concentrate is present, the evaporative concentrate having a concentration strength (described further herein) between about 8 and 12 percent. Other such percentage ranges include: about 4 and 12 percent; and about 0.5 and 12 percent.

In some *Morinda citrifolia* compositions of the present invention, *Morinda citrifolia* fruit juice freeze concentrate is present, the freeze concentrate having a concentration strength (described further herein) between about 8 and 12 percent. Other such percentage ranges include: about 4 and 12 percent; and about 0.5 and 12 percent.

One or more *Morinda citrifolia* products can be further combined with other ingredients or carriers (discussed further herein) to produce a pharmaceutical *Morinda citrifolia* product or composition ("pharmaceutical" herein referring to any drug or product designed to improve the health of living organisms such as human beings or mammals, including nutraceutical products) that is also a *Morinda citrifolia* of the present invention. Examples of pharmaceutical *Morinda citrifolia* products may include, but are not limited to, orally administered solutions and intravenous solutions.

Methods of the present invention comprise the administration and/or consumption of *Morinda citrifolia* compositions in amounts that inhibit the HDAC and/or TACE in mammals. It will be understood that specific dosage levels of any compositions that will be administered to any particular patient will depend upon a variety of factors, including the patient's age, body weight, general health, gender, diet, time of administration, route of administration, rate of excretion, drug combination, and the severity of the particular diseases undergoing therapy or in the process of incubation.

Methods of the present invention also include the obtaining of *Morinda citrifolia* compositions and extracts, including *Morinda citrifolia* fruit juice and concentrates thereof. It will be noted that some of the embodiments of the present invention contemplate obtaining the *Morinda citrifolia* fruit juice pre-made. Various methods of the present invention shall be described in more detail further herein.

The following disclosure of the present invention is grouped into subheadings. The utilization of the subheadings is for convenience of the reader only and is not to be construed as limiting in any sense.

### 1. Obtaining Extracts from the Morinda citrifolia Plant For Incorporation into the Compositions of the Present invention

The Indian Mulberry or Noni plant, known scientifically as *Morinda citrifolia* L*.* (*Morinda citrifolia*), is a shrub or small tree. The leaves are oppositely arranged with an elliptic to ovate form. The small white flowers are contained in a fleshy, globose, head-like cluster. The fruits are large, fleshy, and ovoid. At maturity, they are creamy-white and edible, but have an unpleasant taste and odor. The plant is native to Southeast Asia and has spread in early times to a vast area from India to eastern Polynesia. It grows randomly in the wild, and it has been cultivated in plantations and small individual growing plots. The *Morinda citrifolia* flowers are small, white, three to five lobed, tubular, fragrant, and about 1.25 cm long. The flowers develop into compound fruits composed of many small drupes fused into an ovoid, ellipsoid or round, lumpy body, with waxy, white, or greenish-white or yellowish, semi-translucent skin. The fruit contains "eyes" on its surface, similar to a potato. The fruit is juicy, bitter, dull-yellow or yellowish-white, and contains numerous red-brown, hard, oblong-triangular, winged 2-celled stones, each containing four seeds.

When fully ripe, the fruit has a pronounced odor like rancid cheese. Although the fruit has been eaten by several nationalities as food, the most common use of the *Morinda citrifolia* plant was as a red and yellow dye source. Recently, there has been an interest in the nutritional and health benefits of the *Morinda citrifolia* plant, further discussed below.

Processed *Morinda citrifolia* fruit juice can be prepared by separating seeds and peels from the juice and pulp of a ripened *Morinda citrifolia* fruit; filtering the pulp from the juice; and packaging the juice. Alternatively, rather than packaging the juice, the juice can be immediately included as an ingredient in other products. In some embodiments, the juice and pulp can be pureed into a homogenous blend to be mixed with other ingredients. Other processes include freeze-drying the fruit and juice. The fruit and juice can be reconstituted during production of the final juice product. Still other processes include air-drying the fruit and juices, prior to being masticated.

The present invention also contemplates the use of fruit juice and/or puree fruit juice extracted from the *Morinda citrifolia* plant. In a currently preferred process of producing *Morinda citrifolia* fruit juice, the fruit is either hand picked or picked by mechanical equipment. The fruit can be harvested when it is at least one inch (2-3 cm) and up to 12 inches (24-36 cm) in diameter. The fruit preferably has a color ranging from a dark green through a yellow-green up to a white color, and gradations of color in between. The fruit is thoroughly cleaned after harvesting and before any processing, occurs.

The fruit is allowed to ripen or age from 0 to 14 days, with most fruit being held from 2 to 3 days. The fruit is ripened or aged by being placed on equipment so it does not contact the ground. It is preferably covered with a cloth or netting material during aging, but can be aged without being covered. When ready for further processing the fruit is light in color, from a light green, light yellow, white or translucent color. The fruit is inspected for spoilage or for excessively green color and hard firmness. Spoiled and hard green fruit is separated from the acceptable fruit.

The ripened and aged fruit may be placed in containers for processing and transport. In a preferred embodiment of the invention, the aged fruit is placed in plastic lined containers for further processing and transport. The containers of aged fruit may be held from 0 to 120 days. In a preferred embodiment of the invention, the fruit containers are held for 7 to 14 days before processing. The containers can optionally be stored under refrigerated conditions or ambient/room temperature conditions prior to further processing. The fruit is unpacked from the storage containers and may be further processed through a manual or mechanical separator, in which the seeds and peel are separated from the juice and pulp.

The juice and pulp can be packaged into containers for storage and transport. Alternatively, the juice and pulp can be immediately processed into a finished juice product. The containers can be stored in refrigerated, frozen, or room temperature conditions.

The *Morinda citrifolia* juice and pulp are preferably blended in a homogenous blend, after which they may be mixed with other ingredients. The finished juice product is preferably heated and pasteurized at a minimum temperature of 181°F (83°C) or higher up to 212°F (100°C).

Another product manufactured is *Morinda citrifolia* puree and puree juice, in either concentrate or diluted form. Puree is essentially the pulp separated from the seeds and is different from the fruit juice product described herein.

Each product is filled and sealed into a final container. The container may be plastic, glass, or another suitable material that can withstand the processing temperatures. The containers are maintained at the filling temperature or may be cooled rapidly and then placed in a shipping container. The shipping containers are preferably wrapped with a material and in a manner to maintain or control the temperature of the product in the final containers.

The juice and pulp may be further processed by separating the pulp from the juice through filtering equipment. The filtering equipment preferably consists of, but is not limited to, a centrifuge decanter, a screen filter with a size from 0.01 micron up to 2000 microns, more preferably less than 500 microns, a filter press, reverse osmosis filtration, and any other standard commercial filtration devices. The operating filter pressure preferably ranges from 0.1 psig up to about 1000 psig. The flow rate preferably ranges from 0.1 g.p.m. up to 1000 g.p.m., and more preferably between 5 and 50 g.p.m. The wet pulp may be washed and filtered at least once and up to 10 times to remove any juice from the pulp. The wet pulp typically has a fiber content of 10 to 40 percent by weight. The wet pulp is preferably pasteurized at a temperature of 181°F (83°C) minimum and then packed in drums for further processing or made into a high fiber product.

The processed *Morinda citrifolia product* may also exist as a fiber. Still further, the processed *Morinda citrifolia product* may also exist in oil form, such as an oil extract. The *Morinda citrifolia* oil typically includes a mixture of several different fatty acids as triglycerides, such as palmitic, stearic, oleic, and linoleic fatty acids, and other fatty acids present in lesser quantities. In addition, the oil preferably includes an antioxidant to inhibit spoilage of the oil. Conventional food grade antioxidants are preferably used.

The high fiber product may include wet or dry *Morinda citrifolia* pulp, supplemental fiber ingredients, water, sweeteners, flavoring agents, coloring agents, and/or nutritional ingredients. The supplemental fiber ingredients may include plant based fiber products, either commercially available or developed privately. Examples of some typical fiber products are guar gum, gum arabic, soybean fiber, oat fiber, pea fiber, fig fiber, citrus pulp sacs, hydroxymethylcellulose, cellulose, seaweed, food grade lumber or wood pulp, hemicellulose, etc. Other supplemental fiber ingredients may be derived from grains or grain products. The concentrations of these other fiber raw materials typically range from 0 up to 30 percent, by weight, and more preferably from 10 to 30 percent by weight.

The juice and pulp can be dried using a variety of methods. The juice and pulp mixture can be pasteurized or enzymatically treated prior to drying. The enzymatic process begins with heating the product to a temperature between 75°F and 135°F. It is then treated with either a single enzyme or a combination of enzymes. These enzymes include, but are not limited to, amylase, lipase, protease, cellulase, bromelin, etc. The juice and pulp may also be dried with other ingredients, such as those described above in connection with the high fiber product. The typical nutritional profile of the dried juice and pulp is 1 to 20 percent moisture, 0.1 to 15 percent protein, 0.1 to 20 percent fiber, and the vitamin and mineral content.

The filtered juice and the water from washing the wet pulp are preferably mixed together. The filtered juice may be vacuum evaporated to a brix of 40 to 70 and a moisture of 0.1 to 80 percent, more preferably from 25 to 75 percent. The resulting concentrated *Morinda citrifolia* juice may or may not be pasteurized. For example, the juice would not be pasteurized in circumstances where the sugar content or water activity was sufficiently low enough to prevent microbial growth.

The *Morinda citrifolia* plant is rich in natural ingredients. Those ingredients that have been discovered include: (from the leaves): alanine, anthraquinones, arginine, ascorbic acid, aspartic acid, calcium, beta-carotene, cysteine, cystine, glycine, glutamic acid, glycosides, histidine, iron, leucine, isoleucine, methionine, niacin, phenylalanine, phosphorus, proline, resins, riboflavin, serine, beta-sitosterol, thiamine, threonine, tryptophan, tyrosine, ursolic acid, and valine; (from the flowers): acacetin-7-o-beta-d(+)-glucopyranoside, 5,7-dimethyl-apigenin-4'-o-beta-d(+)-galactopyranoside, and 6,8-dimethoxy-3-methylanthraquinone-1-o-beta-rhamnosyl-glucopyranoside; (from the fruit): acetic acid, asperuloside, butanoic acid, benzoic acid, benzyl alcohol, 1-butanol, caprylic acid, decanoic acid, (E)-6-dodeceno-gamma-lactone, (Z,Z,Z)-8,11,14-eicosatrienoic acid, elaidic acid, ethyl decanoate, ethyl hexanoate, ethyl octanoate, ethyl palmitate, (Z)-6-(ethylthiomethyl) benzene, eugenol, glucose, heptanoic acid, 2-heptanone, hexanal, hexanamide, hexanedioic acid, hexanoic acid (hexoic acid), 1-hexanol, 3-hydroxy-2-butanone, lauric acid, limonene, linoleic acid, 2-methylbutanoic acid, 3-methyl-2-buten-1-ol, 3-methyl-3-buten-1-ol, methyl decanoate, methyl elaidate, methyl hexanoate, methyl 3-methylthio-propanoate, methyl octanoate, methyl oleate, methyl palmitate, 2-methylpropanoic acid, 3-methylthiopropanoic acid, myristic acid, nonanoic acid, octanoic acid (octoic acid), oleic acid, palmitic acid, potassium, scopoletin, undecanoic acid, (Z,Z)-2,5-undecadien-1-ol, and vomifol; (from the roots): anthraquinones, asperuloside (rubichloric acid), damnacanthal, glycosides, morindadiol, morindine, morindone, mucilaginous matter, nor-damnacanthal, rubiadin, rubiadin monomethyl ether, resins, soranjidiol, sterols, and trihydroxymethyl anthraquinone-monomethyl ether; (from the root bark): alizarin, chlororubin, glycosides (pentose, hexose), morindadiol, morindanigrine, morindine, morindone, resinous matter, rubiadin monomethyl ether, and soranjidiol; (from the wood): anthragallol-2,3-dimethylether; (from the tissue culture): damnacanthal, lucidin, lucidin-3-primeveroside, and morindone-6beta-primeveroside; (from the plant): alizarin, alizarin-alpha-methyl ether, anthraquinones, asperuloside, hexanoic acid, morindadiol, morindone, morindogenin, octanoic acid, and ursolic acid.

The present invention contemplates utilizing all parts of the M. *citrifolia* plant alone, in combination with each other or in combination with other ingredients. The above listed portions of the M. *citrifolia* plant are not an exhaustive list of parts of the plant to be used but are merely exemplary. Thus, while some of the parts of the M. *citrifolia* plant are not mentioned above (e.g., seed from the fruit, the pericarp of the fruit, the bark or the plant) the present invention contemplates the use of all of the parts of the plant.

Ingredients, components or extracts may be obtained from any part of the *Morinda cifrifolia* plant including leaves, stem, seeds and/or roots. In a preferred embodiment of the invention, extracts may be obtained from the leaves, stem, seeds, and/or roots by first chopping the raw material. Next, an extraction method may be utilized to isolate ingredients of interest. Extraction of ingredients of interest may be accomplished by exposing the raw ingredients to a solvent of choice. In one embodiment of the invention, a hot water extraction method is utilized, at an appropriate temperature to ensure isolation of the desired ingredients. For example, water may be added to the raw materials in a five to one ratio by weight and heated to 95°C. Other solvents may be utilized for the extraction including organic solvents or mixtures of aqueous and organic solvents. Organic solvents are preferably selected from a list comprising ethanol, methanol, and hexane. Moreover, wet pressure and heat process using ordinary autoclave equipment may be applied. Furthermore, treatment processes using cellulose hydrolysis enzyme may be added to aforementioned processes. After removing insoluble components through filtering, if desired, from extract obtained from leaves, stems, seeds and/or roots, solvent is removed and extract of the present invention is obtained. This extract may be pasteurized, if necessary, or concentrated or dried. Drying may be achieved using ordinary spray drying or freeze-drying. The extract may be stored under cooling or freezing conditions.

Moreover, oil may be extracted from seeds. Oil may be obtained by drying, crushing, and squeezing seeds with a press. More oil may be extracted from seed cake residue by extracting the oil utilizing a solvent selected from a list comprising hexane, ethanol, water, other aqueous solvents, or other organic solvent. The oil contains fatty acid such as linoleic acid, oleic acid, palmitic acid and stearic acid in the form of triglycerides.

### 2. Exemplary Ingredients and Forms for the Compositions of the Present Invention

The compositions of the present invention may be formulated into any of a variety of compositions, including orally administered compositions, intravenous solutions, and other products or compositions. As mentioned earlier herein, the compositions can include a variety of ingredients.

Orally administered compositions may take the form of, for example, liquids or beverages, tablets, lozenges, aqueous or oily suspensions, dispersible powders or granules, emulsions, syrups, or elixirs. Compositions intended for oral use may be prepared according to any method known in the art, and such compositions may contain one or more agents such as sweetening agents, flavoring agents, coloring agents, and preserving agents. They may also contain one or more additional ingredients such as vitamins and minerals, etc. Tablets may be manufactured to contain one or more *Morinda cifrifolia* extracts in admixture with non-toxic, pharmaceutically acceptable excipients that are suitable for the manufacture of tablets. These excipients may be, for example, inert diluents, granulating and disintegrating agents, binding agents, and lubricating agents. The tablets may be uncoated or they may be coated by known techniques to delay disintegration and absorption in the gastrointestinal tract and thereby provide sustained action over a longer period. For example, a time delay material such as glyceryl monostearate or glyceryl distearate may be used.

Aqueous suspensions may be manufactured to contain *Morinda citrifolia* extracts in admixture with excipients suitable for the manufacture of aqueous suspensions.

Examples of such excipients include, but are not limited to: suspending agents such as sodium carboxymethyl-cellulose, methylcellulose, hydroxypropylmethycellulose, sodium alginate, polyvinyl-pyrrolidone, gum tragacanth and gum acacia; dispersing or wetting agents such as a naturally-occurring phosphatide like lecithin, or condensation products of an alkylene oxide with fatty acids such as polyoxyethylene stearate, or condensation products of ethylene oxide with long chain aliphatic alcohols such as heptadecaethylene-oxycetanol, or condensation products of ethylene oxide with partial esters derived from fatty acids and a hexitol such as polyoxyethylene sorbitor monooleate, or condensation products of ethylene oxide with partial esters derived from fatty acids and hexitol anhydrides such as polyethylene sorbitan monooleate.

Typical sweetening agents may include, but are not limited to: natural sugars derived from corn, sugar beets, sugar cane, potatoes, tapioca, or other starch-containing sources that can be chemically or enzymatically converted to crystalline chunks, powders, and/or syrups. Also, sweeteners can comprise artificial or high-intensity sweeteners, some of which may include aspartame, sucralose, stevia, saccharin, etc. The concentration of sweeteners may be between from 0 to 50 percent by weight of the composition, and more preferably between about 1 and 5 percent by weight.

Typical flavoring agents can include, but are not limited to, artificial and/or natural flavoring ingredients that contribute to palatability. The concentration of flavors may range, for example, from 0 to 15 percent by weight of the composition.

Coloring agents may include food-grade artificial or natural coloring agents having a concentration ranging from 0 to 10 percent by weight of the composition.

Typical nutritional ingredients may include vitamins, minerals, trace elements, herbs, botanical extracts, bioactive chemicals, and compounds at concentrations from 0 to 10 percent by weight of the composition. Examples of vitamins include, but are not limited to, vitamins A, BI through B12, C; D, E, Folic Acid, Pantothenic Acid, Biotin, etc. Examples of minerals and trace elements include, but are not limited to, calcium, chromium, copper, cobalt, boron, magnesium, iron, selenium, manganese, molybdenum, potassium, iodine, zinc, phosphorus, etc. Herbs and botanical extracts may include, but are not limited to, alfalfa grass, bee pollen, chlorella powder, Dong Quai powder, Echinacea root, Gingko Biloba extract, Horsetail herb, Indian mulberry, Shitake mushroom, spirulina seaweed, grape seed extract, etc. Typical bioactive chemicals may include, but are not limited to, caffeine, ephedrine, L-carnitine, creatine, lycopene, etc.

Ingredients of the present invention may also include one or more carrier agents (for example, water) known or used in the art. Examples of other ingredients may include, but are not limited to: artificial flavoring, other natural juices or juice concentrates such as a natural grape juice concentrate or a natural blueberry juice concentrate. The ingredients to be utilized in the compositions of the present invention may include any that are safe for internalizing into the body of a mammal.

Favorably, this invention provides a method of inhibiting HDAC and/or TACE with a *Morinda citrifolia*-based formulation without any significant tendency to cause undesirable side effects.

The present invention features a unique formulation and method of administering the same to inhibit HDAC and/or TACE, by providing a nutraceutical composition or treatment formulated with one or more processed *Morinda citrifolia* products derived from the Indian Mulberry plant. The *Morinda citrifolia* product is incorporated into various carriers or nutraceutical compositions suitable for *in vivo* treatment of a patient. For instance, the nutraceutical formulation may be ingested orally, introduced via an intravenous injection or feeding system, or otherwise internalized as is appropriate and directed.

The nutraceutical composition of the present invention comprises one or more of a processed *Morinda citrifolia* product present in an amount by weight between about 0.01 and 100 percent by weight, and preferably between 0.01 and 95 percent by weight. Several exemplary embodiments of formulations are provided below. However, these are only intended to be exemplary, as one ordinarily skilled in the art will recognize other formulations or compositions comprising the processed *Morinda citrifolia* product.

The processed *Morinda citrifolia* product is the active ingredient or contains one or more active ingredients, such as quercetin, rutin, scopoletin, octoanoic acid, potassium, vitamin C, terpenoids, alkaloids, anthraquinones (such as nordamnacanthal, morindone, rubiandin, B-sitosterol, carotene, vitamin A, flavone glycosides, linoleic acid, Alizarin, amino acids, acubin, L-asperuloside, caproic acid, caprylic acid, ursolic acid, and a putative proxeronine and others, for inhibiting HDAC and/or TACE. Active ingredients may be extracted utilizing aqueous or organic solvents including various alcohol or alcohol-based solutions, such as methanol, ethanol, and ethyl acetate, and other alcohol-based derivatives using any known process in the art. The active ingredients of quercetin and rutin are present in amounts by weight ranging from 0.01 - 10 percent of the total formulation or composition. These amounts may be concentrated as well into a more potent concentration in which they are present in amounts ranging from 10 to 100 percent.

The nutraceutical composition comprising *Morinda citrifolia* may be prepared using any known means in the art. In addition, since the nutraceutical composition will most likely be consumed orally, it may contain one or more agents selected from the group consisting of sweetening agents, flavoring agents, coloring agents, preserving agents, and other medicinal agents as directed.

The present invention further features a method of administering a nutraceutical composition comprising one or more processed *Morinda citrifolia* products to inhibit HDAC and/or TACE by providing a nutraceutical composition or treatment formulated The method for administering a nutraceutical, or the method for inhibiting HDAC and/or TACE, comprises the steps of (a) formulating a nutraceutical composition comprising in part a processed *Morinda citrifolia* product present in an amount between about 0.01 and 95 percent by weight, wherein the composition also comprises a carrier, such as water or purified water, and other natural or artificial ingredients; (b) introducing the nutraceutical composition into the body, such that the processed *Morinda citrifolia* product is sufficiently internalized; (c) repeating the above steps as often as necessary to provide an effective amount of the processed *Morinda citrifolia* product to the body of the patient to inhibit HDAC and/or TACE.

The step of introducing the nutraceutical composition into the body comprises one of ingesting the composition orally. Ingesting the nutraceutical orally means the nutraceutical composition may be formulated as a liquid, gel, solid, or some other type that would allow the composition to be quickly digested and concentrated within the body. It is important to note that the step of administering the nutraceutical composition should be carried out in an effective manner so that the greatest concentration of nutraceutical composition, and particularly the processed *Morinda citrifolia* product, is internalized and absorbed into the patient's body. In one embodiment, the nutraceutical composition is administered by taking between 1 teaspoon and 2 oz., and preferably 2 oz., of the nutraceutical composition every two hours each day, or at least twice a day. In addition, the nutraceutical composition is to be taken on an empty stomach, meaning at a period of time at least two hours prior to consumption of any food or drink. Following this, the nutraceutical composition is sufficiently allowed to absorb into the tissues of the body. Of course, one ordinarily skilled in the art will recognize that the amount of composition and frequency of use may vary from individual to individual. For example, the invention contemplates the administration of up to 10 ounces. for each administration.

In another method of the present invention, a takes at least one (1) ounce of Formulation One in the morning on an empty stomach, and at least one (1) ounce at night on an empty stomach, just prior to retiring to bed. In another method of the present invention, a person diagnosed with or experiencing depression takes at least one ounce of Formulation Two twice a day. In addition, the step of administering the nutraceutical composition may include injecting the composition into the body using an intravenous pump.

The following compositions or formulations represent some of the preferred embodiments contemplated by the present invention.

**Formulation One**

| Ingredients | Percent by Weight |
|---|---|
| | |
| *Morinda citrifolia* fruit juice | 100% |

**Formulation Two**

| Ingredients | Percent by Weight |
|---|---|
| | |
| *Morinda citrifolia* fruit juice | 85-99.99% |
| Water | 0.1-15% |

**Formulation Three**

| Ingredients | Percent by Weight |
|---|---|
| | |
| *Morinda citrifolia* fruit juice | 85-99.99% |
| Other fruit juices | 0.1-15% |

**Formulation Four**

| Ingredients | Percent by Weight |
|---|---|
| | |
| *Morinda citrifolia* fruit juice | 50-90% |
| Water | 0.1-50% |
| Other fruit juices | 0.1-30% |

**Formulation Five**

| Ingredients | Percent by Weight |
|---|---|
| | |
| *Morinda citrifolia* extract | 100% |

**Formulation Six**

| Ingredients | Percent by Weight |
|---|---|
| | |
| *Morinda citrifolia* extract | 50-90% |
| Water | 0.1-50% |

**Formulation Seven**

| Ingredients | Percent by Weight |
|---|---|
| | |
| *Morinda citrifolia* extract | 50-90% |
| Other fruit juices | 0.1-30% |

**Formulation Eight**

| Ingredients | Percent by Weight |
|---|---|
| | |
| *Morinda citrifolia* extract | 50-90% |
| Water | 0.1-50% |
| Other fruit juices | 0.1-30% |

**Formulation Nine**

| Ingredients | Percent by Weight |
|---|---|
| | |
| *Morinda citrifolia* extract | 0.1-50% |
| Water | 50-99.9% |

### EXAMPLES

The following examples set forth and present the effects of the *Morinda citrifolia* compositions on the inhibition of HDAC and/or TACE. These examples are not intended to be limiting in any way, but are merely illustrative of the beneficial, advantageous, and remedial effects of *Morinda citrifolia* on HDAC and/or TACE inhibition.

### Example One

In the present example, a patient experiencing or diagnosed with and suffering from Huntington 's disease to treat the condition with a nonprescription, over-the-counter preparation. To treat the disease, the individual consumes an identified prescribed amount of a composition containing processed *Morinda citrifolia* fruit juice. The person intermittently consumes the food product containing the processed *Morinda citrifolia* fruit juice until HDAC and/or TACE is inhibited, wherein the Huntington's disease is reduced or eliminated.

### Example Two

In the present example, a patient experiencing or diagnosed with and suffering from Rheumatoid arthritis desires to treat the condition with a nonprescription, over-the-counter preparation. To treat the neurodegenerative disorder, the individual consumes an identified prescribed amount of a composition containing processed *Morinda citrifolia* fruit juice. The person intermittently consumes the food product containing the processed *Morinda citrifolia* fruit juice until HDAC and/or TACE is sufficiently inhibited, wherein the Rheumatoid arthritis is reduced or eliminated.

### Example Three

The following illustrates results obtained from performing biochemical assays on embodiments of *Morinda citrifolia* fruit juice concentrates of the present invention. Note that "TNJ" refers to *Morinda citrifolia* juice processed according to this invention and commercially available as TAHITIAN NONI ® juice, and that "TNCMP1" refers to *Morinda citrifolia* evaporative concentrate. The percentage of concentration represents the concentration strength of the particular concentrate tested; that is, the strength of concentration relative to the *Morinda citrifolia* fruit juice from which the concentrate was obtained. The percentage of inhibition is the percent by which the HDAC and/or TACE enzyme was inhibited.

**Histone Deacetylase**

| **Test Compound** | **Source of Enzyme** | **Sample Size** | **Concentration of Test Compound** | **Percent Inhibition** |
|---|---|---|---|---|
| TNJ | Rat | 2 | 10% | 88 |
| | | 2 | 5% | 75 |
| | | 2 | 1% | 17 |
| TNCMP1 | Rat | 2 | 5% | 101 |
| | | 2 | 1% | 96 |
| | | 2 | 0.50% | 77 |

### Example Four

The following illustrates results obtained from performing biochemical assays on embodiments of *Morinda citrifolia* fruit juice concentrates of the present invention. Note that "TNJ" refers to *Morinda citrifolia* juice processed according to this invention and commercially available as TAHITIAN NONI ® juice, and that "TNCMP1" refers to *Morinda citrifolia* evaporative concentrate. The percentage of concentration represents the concentration strength of the particular concentrate tested; that is, the strength of concentration relative to the *Morinda citrifolia* fruit juice from which the concentrate was obtained. The percentage of inhibition is the percent by which the HDAC and/or TACE enzyme was inhibited.

**Peptidase, Tumor Necrosis Factor or Converting Enzyme**

| **Test Compound** | **Source of Enzyme** | **Sample Size** | **Concentration of Test Compound** | **Percent Inhibition** |
|---|---|---|---|---|
| TNJ | Hum | 2 | 10% | 99 |
| | | 2 | 5% | 90 |
| | | 2 | 1% | 56 |
| TNCMP1 | Hum | 2 | 10% | 96 |
| | | 2 | 5 | 98 |
| | | 2 | 1% | 94 |

Unless otherwise indicated, any numbers expressing quantities of ingredients, reaction conditions, and so forth present in the specification or any claims or drawings are to be understood as being modified in all instances by the term "about." Accordingly, unless indicated to the contrary, the numerical parameters set forth herein are approximations that may vary depending upon the desired properties sought to be obtained by the present invention. At the very least, each numerical parameter should at least be construed in light of the number of reported significant digits and by applying ordinary rounding techniques.

Notwithstanding that any numerical ranges and parameters that set forth the broad scope of the invention are approximations, the numerical values set forth in the specific examples are reported as precisely as possible. Any numerical value, however, inherently contains certain errors necessarily resulting from the standard deviation found in their respective testing measurements.

While illustrative embodiments of the invention have been described herein, the present invention is not limited to the various preferred embodiments described herein, but includes any and all embodiments having modifications, omissions, combinations, adaptations, and/or alterations as would be appreciated by those in the art based on the present disclosure. The limitations in any claims are to be interpreted broadly based on the language employed in the claims and not limited to examples described herein, which examples are to be construed as non-exclusive. For example, in the present disclosure, the term "preferably" should be construed as meaning "preferably, but not limited to."

The present invention may be embodied in other specific forms without departing from its spirit or essential characteristics. The described embodiments are to be considered in all respects only as illustrative and not restrictive.

## Claims

1. A formulation adapted to inhibit Histone Deacetylase and/or Tumor Necrosis Factor Converting Enzyme comprising: at least one processed *Morinda citrifolia* product present in an amount by weight between 0.1 and 99 percent.

2. The formulation of claim 1, wherein said *Morinda citrifolia* product is used with a carrier medium.

3. The formulation of claim 1, wherein said processed *Morinda citrifolia* product comprises a processed *Morinda citrifolia* product selected from a group comprising: extract from the leaves of *Morinda citrifolia*, leaf hot water extract present in an amount by weight between 0.1 and 50 percent, processed *Morinda citrifolia* leaf ethanol extract present in an amount by weight between 0.1 and 50 percent, processed *Morinda citrifolia* leaf steam distillation extract present in an amount by weight between 0.1 and 50 percent, *Morinda citrifolia* fruit juice, *Morinda citrifolia* extract, *Morinda cifrifolia* dietary fiber, *Morinda citrifolia* puree juice, *Morinda citrifolia* puree, *Morinda citrifolia* fruit juice concentrate, *Morinda citrifolia* puree juice concentrate, freeze concentrated *Morinda citrifolia* fruit juice, and evaporated concentration of *Morinda citrifolia* fruit juice.

4. The formulation of claim 1, further comprising an active ingredient selected from a group comprising quercetin, rutin, scopoletin, octoanoic acid, potassium, vitamin C, terpenoids, alkaloids, anthraquinones, nordamnacanthal, morindone, rubiandin, B-sitosterol, carotene, vitamin A, flavone glycosides, linoleic acid, Alizarin, amino acids, acubin, L-asperuloside, caproic acid, caprylic acid, ursolic acid, and putative proxeronines.

5. The formulation of claim 1, wherein said formulation is administered to a patient by a method selected from a list comprising orally, intravenously, and systemically.

6. The formulation of claim 1, further comprising an ingredient selected from the group comprising processed *Morinda citrifolia* products, food supplements, dietary supplements, other fruit juices, other natural ingredients, natural flavorings, artificial flavorings, natural sweeteners, artificial sweeteners, natural coloring, and artificial coloring.

7. A formulation comprising:
a *Morinda citrifolia* product present in an amount by weight between 0.1 and 99 percent, wherein the formulation is adapted to inhibit Histone Deacetylase and/or Tumor Necrosis Factor Converting Enzyme.

8. A method to inhibit Histone Deacetylase and/or Tumor Necrosis Factor Converting Enzyme in mammals comprising the step of:
administering a formulation containing at least one processed *Morinda citrifolia* product present in an amount by weight between 0.1 and 99 percent.

9. The method of claim 8, wherein two ounces of the formulation is administered twice daily.

10. The method of claim 8, wherein said *Morinda citrifolia* product is administered with a carrier medium.

11. The method of claim 8, wherein said processed *Morinda citrifolia* product comprises a processed *Morinda citrifolia* selected from a group consisting of:
extract from the leaves of *Morinda citrifolia,* leaf hot water extract present in an amount by weight between 0.1 and 50 percent, processed *Morinda citrifolia* leaf ethanol extract present in an amount by weight between 0.1 and 50 percent, processed *Morinda citrifolia* leaf steam distillation extract present in an amount by weight between 0.1 and 50 percent, *Morinda citrifolia* fruit juice, *Morinda citrifolia* extract, *Morinda citrifolia* dietary fiber, *Morinda citrifolia* puree juice, *Morinda citrifolia* puree, *Morinda citrifolia* fruit juice concentrate, *Morinda citrifolia* puree juice concentrate, freeze concentrated *Morinda citrifolia* fruit juice, and evaporated concentration of *Morinda citrifolia* fruit juice.

12. The method of claim 8, wherein the formulation comprises at least one active ingredient selected from a group consisting of quercetin, rutin, scopoletin, octoanoic acid, potassium, vitamin C, terpenoids, alkaloids, anthraquinones, nordamnacanthal, morindone, rubiandin, B-sitosterol, carotene, vitamin A, flavone glycosides, linoleic acid, Alizarin, amino acids, acubin, L-asperuloside, caproic acid, caprylic acid, ursolic acid, and putative proxeronines.

13. The method of claim 8, wherein the formulation further comprising at least one other ingredient selected from the group consisting of processed *Morinda citrifolia* products, food supplements, dietary supplements, other fruit juices, other natural ingredients, natural flavorings, artificial flavorings, natural sweeteners, artificial sweeteners, natural coloring, and artificial coloring.

14. The method of claim 8, further comprising the step of concurrently administering said formulation with another medication designed to improve Histone Deacetylase and/or Tumor Necrosis Factor Converting Enzyme inhibition and associated conditions, wherein said formulation increases the efficacy of said medication.

15. The method of claim 8, wherein said formulation is administered in an amount between 1 teaspoon and 2 ounces at least twice daily on an empty stomach each day.

16. A method of treating mammals comprising:
administering a formulation containing a processed *Morinda citrifolia* product present in an amount by weight between 0.1 and 99 percent, wherein the formulation is adapted to affect a mammal in a way selected from a list consisting of: inhibiting Histone Deacetylase, preventing a complication of a primary disorder in patients wherein said complication results from Histone Deacetylase activity, treating a primary disorder in patients wherein said disorder results from Histone Deacetylase activity, preventing a primary disorder in patients wherein said disorder results from Histone Deacetylase activity, inhibiting Tumor Necrosis Factor Converting Enzyme, treating Huntington's Disease, preventing Huntington's Disease, treating Rheumatoid arthritis, and preventing Rheumatoid arthritis.

17. A formulation for treating mammals comprising:
a processed *Morinda citrifolia* product present in an amount by weight between 0.1 and 99 percent, wherein the formulation is adapted to affect a mammal in a way selected from a list consisting of: inhibiting Histone Deacetylase, preventing a complication of a primary disorder in patients wherein said complication results from Histone Deacetylase activity, treating a primary disorder in patients wherein said disorder results from Histone Deacetylase activity, preventing a primary disorder in patients wherein said disorder results from Histone Deacetylase activity, inhibiting Tumor Necrosis Factor Converting Enzyme, treating Huntington's Disease, preventing Huntington's Disease, treating Rheumatoid arthritis, and preventing Rheumatoid arthritis.
